# EUROPEAN PATENT SPECIFICATION

(11) **EP 0 432 949 B1**
(45) Date of publication and mention of the grant of the patent: **29.05.1996**
(21) Application number: 90313078.9
(22) Date of filing: 30.11.1990
(51) Int. Cl.: C07C 43/13, C07C 41/01, C08G 65/32

(54) **Production of polyalkylene glycol ethers**
Herstellung von Polyalkylenglykoläthern
Préparation d'éthers polyalkylène-glycols

(30) Priority: 07.12.1989 GB 8927682
(43) Date of publication of application: 19.06.1991
(73) Proprietor: IMPERIAL CHEMICAL INDUSTRIES PLC, London SW1P 3JF (GB)
(72) Inventor: Cowling, Ronald, Sale, Cheshire M33 3GW (GB)
(74) Representative: Roberts, Jonathan Winstanley

(56) References cited:
- EP-A- 0 092 463
- DE-A- 2 945 918
- DE-A- 3 224 033
- US-A- 4 088 700

## Description

THIS INVENTION relates to the production of polyalkylene glycol ethers.

Polyethylene glycol mono-alkyl or alkenyl ethers for example adducts of C₈ to C₂₂ alcohols with ethylene oxide are known substances useful as surfactants and as intermediates. They are normally made by condensing ethylene oxide with the alcohol using acidic or preferably basic substances as catalysts.

It is however found that the -OH groups of some especially the branched and particularly the alpha-branched alcohols are less reactive than the -OH groups of the ethoxylates. Thus in such cases once a first molecule of alkylene oxide has reacted with a molecule of the alcohol further alkylene oxide molecules become added to that molecule in preference to unreacted alcohol molecules. Consequently the products tend to contain unreacted alcohol and a wide range of numbers of alkylene oxide groups in the alkoxylated alcohol molecules.

For some purposes a more homogeneous product is required, and if other derivatives, for example alcohol ether sulphates, are to be produced from alkyl ethers of polyethylene glycol this may be particularly important.

It has been proposed in US Patents 4,088,700 and 4,484,009 (Equivalent to EP 0092463 A) to decompose dioxolanes to alkyl ethers of ethylene glycol by hydrogenolysis. However, the former process typically gives substantial yields of ethylene glycol dialkyl ethers as by-products which represents a waste of materials, and the dialkyl ethers are difficult to remove and may degrade the performance of surfactants made from the alcohol monoethoxylates by further alkoxylation. The problem may also occur if the latter process is attempted with the higher homologues. If the products are to be further alkoxylated the alkoxylated materials still have a statistical distribution of alkylene glycol residues which, though better than the products of alkoxylation of alcohols can be improved further in the present invention. Specification DE 3224033 A (Equivalent to US 4479017) describes the catalytic hydrogenation of especially formaldehyde acetals, which cannot undergo side reaction via a vinyl ether intermediate, particularly ether terminated formaldehyde acetals, which may include a 1,3 dioxalane ring system to ethers.

It may also be desired to form alkyl or alkenyl ethers of polyalkylene glycols in which higher glycol residues, for example propylene and/or butylene glycol residues are present. This may also be accomplished in accordance with this invention.

This invention comprises a process of producing a compound of formula R X OH in which R is an alkyl or alkenyl group having 8 to 22 and preferably 9 to 20 carbon atoms X is a residue of a polyalkylene glycol comprising at least two alkylene glycol residues, for example a polyethylene glycol residue for example a group of formula (OC₂H₄)ₙ in which n is at least 2 and preferably at most 10 which comprises reacting an aldehyde of formula R¹CHO in which R¹ is an alkyl or alkenyl group having one less carbon atom than R with a compound of formula H X OH which is preferably present in a molar ratio of 2.0 to 20, preferably 3 to 12 and more preferably 4 to 10 to the aldehyde to form an adduct and hydrogenating the adduct, suitably in the presence of excess compound H X OH present during the formation of the adduct to produce the compound of formula R X OH. R and R¹ are preferably alkyl groups. The polyalkylene glycol residue suitably comprises n ethylene glycol and/or propylene glycol residues.

The process is preferably carried out in two sequential stages, hydrogen and preferably a noble metal catalyst being introduced only after substantial adduct formation has occurred. However, the stages may be combined, especially if an acid and a noble metal catalyst are present with the aldehyde and polyalkylene glycol and hydrogen is present.

It is believed that the adduct may comprise compounds of formula 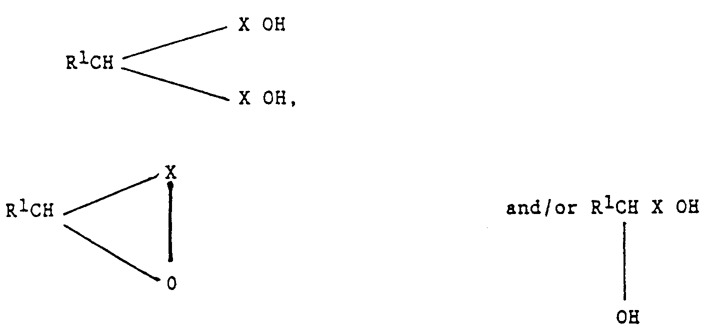
Any of the compound H X OH over the stoichiometric amount which is largely present in the product may be separated for example by vacuum or steam stripping or liquid/liquid extraction for example with water. In some cases it may be desired to use the product in a formulation in which it is desired that the compound H X OH, for example polyethylene glycol should also be present, and in this case separation may not be necessary.

The higher polyalkylene glycols, for example of molecular weight above about 200, are usually obtained as mixtures of compounds of a specified average molecular weight rather than as individual compounds. The value n above should be taken as referring to the average number of groups X and may thus not be an integer.

When, X is (OC₂H₄)₂ the product is suitably separated from diethylene glycol and may then be converted into the sulphate. This is of value in shampoo formulations and contains little alcohol sulphate (which is a skin irritant) as compared with similar substances produced by ethoxylating alcohols and sulphating the ethoxylate.

The formation of the adduct is suitably carried out by contacting the aldehyde with excess polyalkylene glycol in the presence of an acid catalyst, for example HCl, H₂SO₄ or a sulphonic acid, for example toluene sulphuric acid, but preferably phosphoric acid for example in a concentration based on the reactants of 0.01 to 1% by weight, suitably at a temperature of 50 to 250°C and preferably 70 to 150°C, suitably with removal of water. The pressure is not critical but pressures of 0.005 to 20 bars absolute for example may be used.

Reduction may be carried out on the crude adduct. It is desirable that phosphoric acid catalyst should be present during the reduction step and in the adduct forming step. Reduction may be effected by contacting the adduct with hydrogen for example at a pressure of 10 to 500 and preferably 15 to 50 bars absolute and at a temperature of for example 150 to 300°C and preferably 200 to 250°C with a hydrogenation catalyst which suitably comprises a noble metal of group 8 of the periodic table, for example platinum, rhodium or preferably palladium which is preferably supported on a porous support for example a carbon or alpha alumina support. The noble metal may be present in an amount of 1 to 1000 and preferably 3 to 200 and more preferably 5 to 40 parts per million by weight based on the total material present in the reaction if the reaction is carried out as a batch reaction. Excessive levels of catalyst may result in unnecessary production of alkane by products and a consequential reduction in yields of the desired products. If the noble metal is on a support it may be present as 0.1 to 20% and preferably 1 to 10% by weight of the support. If desired, an antioxidant may be present to prevent to conversion of acetals to esters but it is preferred to exclude oxygen from the process in which case the antioxidant is unnecessary.

Removal of the noble metal catalyst may be carried out by for example filtration and any acid present may be neutralised if desired. Noble metal catalyst can be re-used several times.

Recovered H X OH can be recycled to the process if desired. Subsequent conversion of the compound of formula R X OH to a sulphate with SO₃ or alkoxylation of the said compound with for example ethylene and/or propylene oxide may be carried out in known manner.

### Example 1

### Preparation of Diethylene Glycol C₁₃/C₁₅-alkyl Ether

### (a) Adduct formation

The aldehyde raw material was a mixture of C₁₃- and C₁₅ - aldehydes in a ratio of 2:1 (approx), and of straight and branched chain aldehdyes in a ratio of 1:1 (approx). The aldehyde (110g, 0.5M) was mixed with diethylene glycol (424.5g, 4M), and phosphoric acid (0.48g, 100% str, 900 ppm w/w). The stirred mixture was subjected to a reduced pressure of approximately 0.02 bars absolute with a slow stream of nitrogen passing through, and the temperature was raised to 85-90°C. After 2 hr at this temperature the mixture was heated to 130°C over 1 hr and held at that temperature for 2 hr. Analysis of the cooled, homogeneous solution by gas chromatography showed that approximately 5% of the aldehyde remained unreacted.

### (b) Formation of the diethylene glycol ether

The total mixture from stage (a) was charged to a one litre stainless steel autoclave together with 5% Pd/carbon catalyst paste (0.375g, 17 ppm Pd w/w) and hydroquinone (0.0161g, 30 ppm w/w). The autoclave was flushed with hydrogen, and then sealed. The stirred mixture was then heated to 225°C over 1 hr (approx). Hydrogen pressure was then applied to a total pressure of 28 bars gauge which was maintained for 12 hr. The autoclave was cooled to ambient temperature, the pressure released, and the product mixture was discharged.

The product mixture was filtered to remove the Pd/carbon catalyst, and the filtrate separated into two phases. The upper layer was stripped of lower boiling impurities by subjecting it to a pressure of approximately 0.02 bar absolute with a slow stream of nitrogen passing through, and with the temperature gradually being raised to 200°C (approx). The cooled, clear, product was analysed by gas chromatography which gave the composition:-

| | |
|---|---|
| Diethylene glycol C_{13/15}-alkyl ether | 92% |
| Diethylene glycol di_(C_{13/15}-alkyl) ether | 7% |
| Di-(C_{13/15}-alkyl) ether | < 1% |
| C_{13/15}-alcohol | < 1% |
| C_{13/15}-aldehyde | < 1% |
| C_{12/15}-alkanes | < 1% |
| Diethylene glycol | < 1% |

The yield of diethylene glycol C_{13/15}-alkyl ether was 116g expressed as pure product
= 75% theory based on aldehyde consumed

### Example 2

### Preparation of Diethylene glycol C_{9/11}-alkyl Ether

### Adduct formation

(a) The aldehyde raw material was a mixture of C₉ and C₁₁ aldehydes in a ratio of 1:2 (approx), and of straight and branched chain aldehydes at a ratio of 1:1 (approx). The aldehyde (71.6g, 0.4M) was mixed with diethylene glycol (424.5g, 4M) and phosphoric acid (0.446g, 100% str, 900 ppm w/w). The stirred mixture was subjected to a reduced pressure of approximately 0.13 bars absolute with a slow stream of nitrogen passing through, and the temperature was raised to 70°C. After 1 hr at this temperature the mixture was slowly heated to 130°C, and held for 2 hr at that temperature. Analysis of the cooled, homogeneous solution by gas chromatography showed that approximately 25% of the aldehyde remained unreacted.

### Formation of the diethylene glycol ether

The total mixture from stage (a) was charged to a one litre stainless steel autoclave together with 5% Pd/C catalyst paste (3.47g,170 ppm Pd w/w) and hydroquinone (0.0149g, 30 ppm w/w). The autoclave was flushed with hydrogen, and sealed. The stirred mixture was then raised to a temperature of 225°C over 1 hr (approx). Hydrogen pressure was then applied to a total pressure of 103 bars gauge which was maintained for 12 hr. The autoclave was cooled, the pressure released, and the product was discharged.

The product mixture was filtered to remove Pd/carbon catalyst, and to give a homogeneous filtrate. The filtrate was mixed with water (100 ml) and the two layers were separated. The top layer was shaken with water (50 ml), and the mixture heated to effect separation and removal of the product. The separated product was dried by heating under reduced pressure, and analysed by gas chromatography which gave the composition:-

| | |
|---|---|
| Diethylene glycol C_{9/11} -alkyl ether | 91-92% |
| Diethylene glycol di-(C_{9/11}-alkyl) ether | 5% |
| Di-(C_{9/11}-alkyl) ether | < 1% |
| C_{9/11}-alcohol | < 1% |
| C_{9/11} - aldehyde | < 1% |
| Alkanes | < 1% |
| Diethylene glycol | ∼ 1% |

It appears that most of the unreacted aldehyde from the first stage reacted in the second stage.

### Example 3

### Preparation of Triethylene Glycol C_{13/15}-alkyl Ether

### (a) Adduct formation

The aldehyde raw material was as described in Example 1. The aldehyde (66g, 0.3M) was mixed with triethylene glycol (450.5g, 3M) and phosphoric acid (0.465g, 100% str, 900 ppm w/w) and the adduct was formed as described in Example 1. Analysis of the cooled, homogeneous solution by gas chromatography showed that approximately 1% of the aldehyde remained unreacted.

### (b) Formation of the triethylene glycol ether

The total mixture from Stage (a) was charged to a one litre stainless steel autoclave together with 5% Pd/carbon catalyst paste (3.62g, 170 ppm Pd w/w), and hydroquinone (0.0155g, 30 ppm w/w). The autoclave was flushed with hydrogen, and then sealed. The stirred mixture was raised to a temperature of 225°C over 1 hr (approx). Hydrogen pressure was then applied to a total pressure of 103 bars gauge which was maintained for 12 hr. The autoclave was cooled to ambient temperature, the pressure released, and the product mixture was discharged.

The product mixture was filtered to remove Pd/Carbon catalyst, and to give a homogeneous filtrate. The filtrate was mixed with water (100 ml), and the two layers separated. The top layer containing the product was shaken with water (50 ml), and the mixture heated to effect separation and removal of the product. The separated product was dried by heating under reduced pressure, and analysed by gas chromatography which gave the composition:-

| | |
|---|---|
| Triethylene glycol C_{13/15}-alkyl ether | 93% |
| Triethylene glycol di-(C_{13/15}-alkyl) ether | 2% |
| Di- (C_{13/15}-alkyl) ether | < 1% |
| C_{13/15} - alcohol | < 1% |
| C_{13/15} - aldehyde | < 1% |
| C_{12/15}- alkanes | 2-3% |
| Triethylene glycol | < 1% |

w/w means by weight
str. means strength
hr means hour

## Claims

1. A process of producing a compound of formula R X OH in which R is an alkyl or alkenyl group having 8 to 22 carbon atoms X is a residue of a polyalkylene glycol comprising at least two alkylene glycol residues which comprises reacting an aldehyde of formula R¹CHO in which R¹ is an alkyl or alkenyl group having one less carbon atom than R with a compound of formula H X OH to form an adduct and hydrogenating the adduct to produce the compound of formula R X OH.

2. A process as claimed in claim 1 in which R has 9 to 20 carbon atoms and, X is of formula (OC₂H₄)ₙ where n is 2 to 10.

3. A process as claimed in claim 1 or 2 in which the compound of formula H X OH is present in a molar ratio of 3 to 12 to the aldehyde before the adduct is formed and the adduct is hydrogenated in the presence of the excess polyethylene glycol.

4. A process as claimed in any preceding claim in which the adduct formation and hydrogenation are carried out in two separate stages.

5. A process as claimed in any preceding claim in which R and R¹ are alkyl groups having 9 to 20 and 8 to 19 carbon atoms respectively.

6. A process as claimed in any preceding claim in which the adduct is formed in the presence of phosphoric acid which is also present in the reduction step.

7. A process as claimed in any preceding claim in which the compound H X OH is diethylene glycol and in which the product is separated from diethylene glycol and converted into its sulphate.

8. A process as claimed in any preceding claim in which the adduct is formed in the presence of an acid catalyst in a concentration of 0.01 to 1% by weight at a temperature of 50 to 250°C with removal of water and reduction is carried out by contacting the crude adduct with hydrogen at a pressure of 10 to 500 Bar (1 to 50 MPa) absolute in the presence of a hydrogenation catalyst at a temperature of 150 to 300°C.

9. A process as claimed in claim 8 in which the hydrogenation catalyst is a noble metal of group 8 of the periodic table in an amount of 1 to 1000 parts per million by weight.

10. A process as claimed in any preceding claim in which oxygen is excluded.

11. A process as claimed in any one of claims 1-6 or 8-10 in which the compound of formula R X OH is reacted with ethylene and/or propylene oxide to produce a more highly alkoxylated derivative.

## Patentansprüche

1. Verfahren zur Herstellung einer Verbindung mit der Formel R X OH, in der R für eine Alkyl- oder Alkenyl-Gruppe mit 8 bis 22 Kohlenstoffatomen steht und X ein Rest eines Polyalkylenglycols ist, der mindestens zwei Alkylenglycol-Reste aufweist, bei dem ein Aldehyd mit der Formel R¹CHO, in der R¹ für eine Alkyl- oder Alkenyl-Gruppe steht, die ein Kohlenstoffatom weniger als R hat, mit einer Verbindung mit der Formel H X OH unter Bildung eines Addukts umgesetzt und das Addukt zur Bildung der Verbindung mit der Formel R X OH hydriert wird.

2. Verfahren nach Anspruch 1, wobei R 9 bis 20 Kohlenstoffatome hat und X die Formel (OC₂H₄)ₙ aufweist, in der n 2 bis 10 ist.

3. Verfahren nach Anspruch 1 oder 2, wobei die Verbindung mit der Formel H X OH in einem Molverhältnis von 3 bis 12 bezüglich des Aldehyds vorliegt, bevor das Addukt gebildet wird, und das Addukt in Gegenwart des überschüssigen Polyethylenglycols hydriert wird.

4. Verfahren nach einem der vorhergehenden Ansprüche, wobei die Adduktbildung und die Hydrierung in zwei unterschiedlichen Stufen durchgeführt werden.

5. Verfahren nach einem der vorhergehenden Ansprüche, wobei R und R¹ Alkyl-Gruppen mit 9 bis 20 bzw. 8 bis 19 Kohlenstoffatomen sind.

6. Verfahren nach einem der vorhergehenden Ansprüche, wobei das Addukt in Gegenwart von Phosphorsäure, die ebenfalls in dem Reduktionsschritt vorhanden ist, gebildet wird.

7. Verfahren nach einem der vorhergehenden Ansprüche, wobei die Verbindung H X OH Diethylenglycol ist und das Produkt von dem Diethylenglycol abgetrennt und in sein Sulfat umgewandelt wird.

8. Verfahren nach einem der vorhergehenden Ansprüche, wobei das Addukt in Gegenwart eines Säurekatalysators in einer Konzentration von 0,01 bis 1 Gew.% bei einer Temperatur von 50 bis 250°C unter Entfernung von Wasser gebildet und die Reduktion durch Kontaktieren des rohen Addukts mit Wasserstoff bei einem Druck von 10 bis 500 bar (1 bis 50 MPa) absolut in Gegenwart eines Hydrierungskatalysators bei einer Temperatur von 150 bis 300°C durchgeführt wird.

9. Verfahren nach Anspruch 8, wobei der Hydrierungskatalysator ein Edelmetall der Gruppe 8 des Periodensystems in einer Menge von 1 bis 1000 Gewichtsteilen pro Million Gewichtsteile ist.

10. Verfahren nach einem der vorhergehenden Ansprüche, wobei Sauerstoff ausgeschlossen ist.

11. Verfahren nach einem der Ansprüche 1 bis 6 oder 8 bis 10, wobei die Verbindung mit der Formel R X OH mit Ethylen- und/oder Propylenoxid zur Herstellung eines höher alkoxylierten Derivats umgesetzt wird.

## Revendications

1. Procédé de production d'un composé de formule R X OH dans laquelle R représente un groupe alkyle ou alcényle ayant 8 à 22 atomes de carbone, X représente un résidu d'un polyalkylène-glycol comprenant au moins deux résidus alkylène-glycol, qui comprend la réaction d'un aldéhyde de formule R¹CHO dans laquelle R¹ représente un groupe alkyle ou alcényle ayant un atome de carbone de moins que R avec un composé de formule H X OH pour former un produit d'addition, et l'hydrogénation du produit d'addition pour produire le composé de formule R X OH.

2. Procédé suivant la revendication 1, dans lequel R a 9 à 20 atomes de carbone et X répond à la formule (OC₂H₄)ₙ dans laquelle n a une valeur de 2 à 10.

3. Procédé suivant la revendication 1 ou 2, dans lequel le composé de formule H X OH est présent en un rapport molaire dudit composé à l'aldéhyde de 3 à 12 avant la formation du produit d'addition, et le produit d'addition est hydrogéné en présence du polyéthylène-glycol en excès.

4. Procédé suivant l'une quelconque des revendications précédentes, dans lequel la formation et l'hydrogénation du produit d'addition sont effectuées en deux étapes distinctes.

5. Procédé suivant l'une quelconque des revendications précédentes, dans lequel R et R¹ représentent des groupes alkyle ayant, respectivement, 9 à 20 et 8 à 19 atomes de carbone.

6. Procédé suivant l'une quelconque des revendications précédentes, dans lequel le produit d'addition est formé en présence d'acide phosphorique qui est également présent dans l'étape de réduction.

7. Procédé suivant l'une quelconque des revendications précédentes, dans lequel le composé H X OH est le diéthylène-glycol et dans lequel le produit est séparé du diéthylène-glycol et transformé en son sulfate.

8. Procédé suivant l'une quelconque des revendications précédentes, dans lequel le produit d'addition est formé en présence d'un catalyseur acide en une concentration de 0,01 à 1 % en poids à une température de 50 à 250°C, avec élimination d'eau, et la réduction est effectuée en mettant en contact le produit brut d'addition avec de l'hydrogène sous une pression absolue de 10 à 500 bars (1 à 50 MPa) en présence d'un catalyseur d'hydrogénation à une température de 150 à 300°C.

9. Procédé suivant la revendication 8, dans lequel le catalyseur d'hydrogénation est un métal noble du Groupe 8 du Tableau Périodique, en une quantité de 1 à 1000 parties par million, en poids.

10. Procédé suivant l'une quelconque des revendications précédentes, dans lequel l'oxygène est exclus.

11. Procédé suivant l'une quelconque des revendications 1 à 6 ou 8 à 10, dans lequel le composé de formule R X OH est amené à réagir avec l'oxyde d'éthylène et/ou l'oxyde de propylène pour produire un dérivé plus fortement alkoxylé.
